# EUROPÄISCHE PATENTANMELDUNG

(11) **EP 4 474 338 A1**
(43) Veröffentlichungstag der Anmeldung: **11.12.2024**
(21) Anmeldenummer: 23177508.1
(22) Anmeldetag: 06.06.2023
(51) Int. Cl.: B67B 7/92, A61B 17/88, B01F 35/71

(54) **AMPULLENÖFFNER MIT MULTIFUNKTIONELLEM KOLBEN**

(71) Anmelder: Heraeus Medical GmbH, 61273 Wehrheim (DE)
(72) Erfinder: VOGT, Sebastian, 61273 Wehrheim (DE); KLUGE, Thomas, 61273 Wehrheim (DE)
(74) Vertreter: Heraeus IP

(57) **Zusammenfassung**

Die Erfindung betrifft eine Vorrichtung (100) zur Abgabe einer Flüssigkeit (202) aus einer Glasampulle (201), aufweisend ein Gehäuse (101), eine in dem Gehäuse angeordnete Halterung (102) zur Aufnahme einer Glasampulle, einen in dem Gehäuse angeordneten Vorsprung (103), der zum Aufbrechen einer in der Halterung aufnehmbaren Glasampulle ausgebildet und eingerichtet ist, einen Auslass (104) zur Abgabe einer Flüssigkeit aus dem Gehäuse, und einen in dem Gehäuse beweglich angeordneten Kolben (105), wobei der Kolben ausgebildet und eingerichtet ist, eine in der Halterung aufnehmbare Glasampulle durch Druck der Glasampulle gegen den Vorsprung aufzubrechen, und die Flüssigkeit aus der Glasampulle aus dem Auslass des Gehäuses zu pumpen.

## Beschreibung

### GEBIET DER ERFINDUNG

Die vorliegende Erfindung betrifft das Gebiet der Medizintechnik, insbesondere Vorrichtungen für die Zubereitung von Knochenzement. Insbesondere werden Vorrichtungen zum Öffnen von Glasampullen beschrieben, welche zur Abgabe einer Flüssigkeit aus solchen Glasampullen geeignet sind. Beispielsweise können durch die hierin beschriebenen Vorrichtungen eine Monomerflüssigkeit an eine Pulverkomponente zur Herstellung eines Polymethylmethacrylat-Knochenzements abgegeben werden. Die hierin beschriebenen Vorrichtungen können allgemein zum Lagern und Öffnen von Glasampullen und zur Abgabe einer darin enthaltenen Flüssigkeit dienen.

### TECHNISCHER HINTERGRUND

die hierin beschriebenen Vorrichtungen können bevorzugt zum Lagern und Öffnen von Ampullen und zum Transfer von Monomerflüssigkeit in vorbefüllte Polymethylmethacrylat-Knochenzementmischsysteme dienen. Weitere Gegenstände der Erfindung sind mit den Zementkomponenten Knochenzementpulver und Monomerflüssigkeit vorbefüllte Polymethylmethacrylat-Knochenzementmischsysteme, welche die Vorrichtung zum Lagern und Öffnen von Ampullen enthalten. Die hierin beschriebenen Vorrichtungen können bevorzugt für sogenannte "Full-Prepacked Mischsysteme" für Polymethylmethacrylat-Knochenzement verwendet werden, welche die benötigten Komponenten zur Herstellung eines Knochenzements in getrennt versiegelten Behältern enthalten.

Polymethylmethacrylat-(PMMA)-Knochenzemente gehen auf die grundlegenden Arbeiten von Charnley zurück. PMMA-Knochenzemente bestehen aus einer flüssigen Monomerkomponente und einer Pulverkomponente. Die Monomerkomponente enthält im Allgemeinen das Monomer Methylmethacrylat und einen darin gelösten Aktivator (N,N-Dimethyl-p-toluidin). Die Pulverkomponente, auch als Knochenzementpulver bezeichnet, weist ein oder mehrere Polymere, die auf Basis von Methylmethacrylat und Comonomeren, wie Styren, Methylacrylat oder ähnlichen Monomeren durch Polymerisation, vorzugsweise Suspensionspolymerisation, hergestellt sind, einen Röntgenopaker und den Initiator Dibenzoylperoxid auf. Beim Vermischen der Pulverkomponente mit der Monomerkomponente entsteht durch Quellung der Polymere der Pulverkomponente im Methylmethacrylat ein plastisch verformbarer Teig, der eigentliche Knochenzementteig. Beim Vermischen der Pulverkomponente mit der Monomerkomponente reagiert der Aktivator N,N-Dimethyl-p-toluidin mit Dibenzoylperoxid unter Bildung von Radikalen. Die gebildeten Radikale initiieren die radikalische Polymerisation des Methylmethacrylates. Mit fortschreitender Polymerisation des Methylmethacrylates erhöht sich die Viskosität des Zementteigs bis dieser erstarrt.

Polymethylmethacrylat-Knochenzemente können in geeigneten Mischbechern mit Hilfe von Spateln durch Vermischen des Zementpulvers mit der Monomerflüssigkeit vermischt werden. Dabei kann es zum Einschluss von Luftblasen im Knochenzementteig kommen, welche die mechanischen Eigenschaften des ausgehärteten Knochenzements negativ beeinflussen können.

Zur Vermeidung von Lufteinschlüssen im Knochenzementteig wurden eine Vielzahl von Vakuum-Zementiersystemen beschrieben, von denen exemplarisch folgende genannt sind: US6,033,105A, US5,624,184A, US4,671,263A, US4,973,168A, US5,100,241A, WO99/67015A1, EP1 020 167A2, US5,586,821A, EP1 016 452A2, DE36 40 279A1, WO94/26403A1, EP1 005 901A2, und US5,344,232A.

Eine Weiterentwicklung in der Zementiertechnik stellen Zementiersysteme dar, in denen sowohl das Zementpulver als auch die Monomerflüssigkeit bereits in separaten Kompartimenten der Mischsysteme verpackt sind und die bevorzugt erst unmittelbar vor der Zementapplikation im Zementiersystem miteinander vermischt werden können. Solche geschlossenen Full-Prepacked-Mischsysteme wurden in den Patenten EP0380867B1, EP0796653B1, EP0692229B1, DE102009031178B1, US5997544B1, US6709149B1, DE69812726B1 und US5588745B1, WO9416951A1, DE19718648A1, EP1741413B1, EP3054880B1, DE102009031178B3, US8662736B2 und EP3093067B1 beschrieben.

Die Monomerflüssigkeit von PMMA-Knochenzement lässt sich vorteilhaft in Glasampullen und auch in Kunststoffverbundbeuteln sowie auch in metallisierten Kunststoffverbundbeuteln über Zeiträume von Jahren lagern. Besonders geeignet sind dabei Glasampullen, weil diese einen Austritt der Monomerflüssigkeit aus der Ampulle durch Diffusion vollständig verhindern.

Zur Entnahme der Monomerflüssigkeit aus einer Glasampulle muss jedoch der Ampullenkopf vom Ampullenkörper abgetrennt und entfernt werden. Zur Öffnung von Glasampullen in geschlossenen Ampullenhaltern wurden eine Reihe von Patentschriften bekannt. Vorrichtungen zum Öffnen von Ampullen wurden in den Schriften DE19532015A1, WO9718031A1, und WÖ2010012114A1 offenbart.

EP2404864B1 offenbart eine Vorrichtung, bei der eine Ampulle in einem Ampullenhalter angeordnet ist und wobei im Bereich des Ampullenhalses der Ampullenhalter elastisch deformierbar ist. Im Bereich des Ampullenkopfs und des Ampullenkörpers ist der Ampullenhalter nicht deformierbar. Durch Knicken des Ampullenhalters über den elastisch deformierbaren Abschnitt des Ampullenhalters, der eine Scharnierfunktion hat, kann der Ampullenkopf gegen die nicht deformierbare Wandung des Ampullenhalters gedrückt werden. Dadurch kann der Ampullenkopf abbrechen und auf ein Sieb fallen. Die Monomerflüssigkeit kann danach aus der geöffneten Ampulle durch das Sieb fließen. Für die Funktion der dort beschriebenen Vorrichtung ist es vorteilhaft, dass diese mit einem mit Monomerflüssigkeit zu versorgenden Mischsystem mechanisch stabil verbindbar ist, weil eine erhebliche Zugbelastung beim Abknickvorgang an der Befestigung zum Mischsystem auftreten kann.

### BEVORZUGTE AUSFÜHRUNGSFORMEN

Die Aufgabe der vorliegenden Erfindung besteht darin, eines oder mehrere der oben geschilderten und weitere Probleme des Stands der Technik zu lösen. Beispielsweise ermöglicht die Erfindung eine einfache und sichere Abgabe einer Flüssigkeit aus einer Glasampulle. Weiterhin liefert die vorliegende Erfindung ein Verfahren zur Abgabe einer Flüssigkeit aus einer Glasampulle.

Diese Aufgaben werden gelöst durch die hierin beschriebenen Verfahren und Vorrichtungen, insbesondere denjenigen, die in den Patentansprüchen beschrieben sind.

Bevorzugte Ausführungsformen der Erfindung werden nachstehend beschrieben.

Ein erster Aspekt betrifft eine Vorrichtung zur Abgabe einer Flüssigkeit aus einer Glasampulle, aufweisend
- ein Gehäuse,
- eine in dem Gehäuse angeordnete Halterung zur Aufnahme einer Glasampulle mit einer Flüssigkeit,
- einen in dem Gehäuse angeordneten Vorsprung, der zum Aufbrechen einer in der Halterung aufnehmbaren Glasampulle ausgebildet und eingerichtet ist,
- einen Auslass zur Abgabe einer Flüssigkeit aus dem Gehäuse, und
- einen in dem Gehäuse beweglich angeordneten Kolben, wobei der Kolben ausgebildet und eingerichtet ist, eine in der Halterung aufnehmbare Glasampulle durch Druck der Glasampulle gegen den Vorsprung aufzubrechen, und die Flüssigkeit aus der Glasampulle aus dem Auslass des Gehäuses zu pumpen.

Eine zweite Ausführungsform beschreibt eine Vorrichtung gemäß der oben beschriebenen ersten Ausführungsform, weiterhin aufweisend ein Ventil, welches eingerichtet ist, Luft von außen in das Gehäuse einströmen zu lassen, um anschließend durch Druck des Kolbens auf die in das Gehäuse eingeströmte Luft eine Flüssigkeit aus einer in der Vorrichtung aufgebrochenen Glasampulle aus dem Gehäuse zu pumpen.

Eine dritte Ausführungsform betrifft eine Vorrichtung gemäß der zweiten Ausführungsform, wobei das Ventil ein Einwegeventil, bevorzugt ein Kugelventil, Lippenventil oder Plattenventil ist.

Eine vierte Ausführungsform betrifft eine Vorrichtung nach einer der vorangehenden Ausführungsformen, weiterhin aufweisend einen Auffangbereich, der in dem Gehäuse angeordnet und zur Aufnahme eines durch Wirkung des Kolbens abgebrochenen Teils einer Glasampulle eingerichtet ist.

Eine fünfte Ausführungsform betrifft eine Vorrichtung nach Ausführungsform 4, weiterhin aufweisend ein Sieb, welches angrenzend an den Auffangbereich angeordnet ist, und eingerichtet ist, Bruchstücke aus einer in der Vorrichtung aufgebrochenen Glasampulle in der Vorrichtung zurückzuhalten.

Eine sechste Ausführungsform betrifft eine Vorrichtung nach einer der vorangehenden Ausführungsformen, wobei die Vorrichtung weiterhin einen Sammelbereich aufweist, um Flüssigkeit aus einer in der Vorrichtung aufgebrochenen Glasampulle aufzunehmen.

Eine siebte Ausführungsform betrifft eine Vorrichtung nach einer der vorangehenden Ausführungsformen, wobei die Vorrichtung zum Mischen von Knochenzement ausgebildet und eingerichtet ist, wobei die Vorrichtung bevorzugt weiterhin eine Pulverkomponente zur Zubereitung eines Knochenzements enthält.

Eine achte Ausführungsform betrifft eine Vorrichtung nach einer der vorangehenden Ausführungsformen, wobei die Vorrichtung ein erstes Ende und ein zweites Ende aufweist, wobei das erste Ende dem zweiten Ende gegenüberliegend angeordnet ist, wobei der Kolben an dem ersten Ende angeordnet ist, und der Auslass an dem zweiten Ende angeordnet ist.

Eine neunte Ausführungsform betrifft eine Vorrichtung nach Ausführungsform 8, wobei der Auslass eine Längsachse der Vorrichtung (L) definiert, wobei die Vorrichtung eingerichtet ist, eine Flüssigkeit entlang dieser Längsachse abzugeben, wobei die Halterung in einem Winkel α von 0° bis 50° zu dieser Längsachse (L) angeordnet ist.

Eine zehnte Ausführungsform betrifft eine Vorrichtung nach einer der vorangehenden Ausführungsformen, wobei der Auslass zur Verbindung mit einem Behälter ausgebildet und eingerichtet ist, wobei der Behälter bevorzugt zur Aufnahme einer Pulverkomponente eines Knochenzements eingerichtet ist.

Eine elfte Ausführungsform betrifft eine Vorrichtung nach einer der vorangehenden Ausführungsformen, wobei die Vorrichtung ausgebildet und eingerichtet ist, mit einem einzigen Hub des Kolbens den gesamten Inhalt einer in der Halterung aufgenommenen Glasampulle aus dem Auslass des Gehäuses zu pumpen.

Eine zwölfte Ausführungsform betrifft eine Vorrichtung nach einer der vorangehenden Ausführungsformen, wobei eine Glasampulle kraftschlüssig, formschlüssig oder stoffschlüssig in der Halterung befestigt oder fixierbar ist, bevorzugt durch Kleben oder Klemmen befestigt oder fixierbar ist.

Eine dreizehnte Ausführungsform betrifft eine Vorrichtung nach einer der vorangehenden Ausführungsformen, wobei die Vorrichtung zum gleichzeitigen Aufbrechen und Abgeben von Flüssigkeit aus zwei Glasampullen ausgebildet und eingerichtet ist.

Eine vierzehnte Ausführungsform betrifft eine Vorrichtung nach einer der vorangehenden Ausführungsformen, wobei in der Halterung eine Glasampulle 201 angeordnet ist, die einen Ampullenhals mit einer ringförmigen Sollbruchstelle aufweist, wobei der Innendurchmesser der Sollbruchstelle bevorzugt 4 bis 7 mm beträgt.

Ein weiterer Aspekt betrifft ein Verfahren zur Abgabe einer Flüssigkeit aus einer Glasampulle mit Hilfe einer oben beschriebenen Vorrichtung, wobei das Verfahren die folgenden Schritte aufweist,
- Fixieren der Glasampulle in einer Halterung der Vorrichtung;
- Aufbrechen der Glasampulle durch Drücken der Glasampulle gegen einen Vorsprung der Vorrichtung mit Hilfe eines Kolbens der Vorrichtung;
- Abgabe der Flüssigkeit aus der aufgebrochenen Glasampulle durch Pumpen mithilfe des Kolbens durch einen Auslass der Vorrichtung.

### AUSFÜHRLICHE BESCHREIBUNG

Zu den hierin beschriebenen Ausführungsformen, deren Elemente ein bestimmtes Merkmal (z.B. ein Material) "aufweisen", oder "umfassen" wird grundsätzlich immer eine weitere Ausführungsform erwogen, in denen das betreffende Element allein aus dem Merkmal besteht, d.h. keine weiteren Bestandteile umfasst. Das Wort "umfassen" oder "umfassend" wird hierin synonym mit dem Wort "aufweisen" oder "aufweisend" verwendet.

Wenn in einer Ausführungsform ein Element mit dem Singular bezeichnet ist, wird ebenfalls eine Ausführungsform erwogen, bei denen mehrere dieser Elemente vorhanden sind. Die Verwendung eines Begriffs für ein Element im Plural umfasst grundsätzlich auch eine Ausführungsform, in welchem nur ein einzelnes entsprechendes Element enthalten ist.

Soweit nicht anders angegeben oder aus dem Zusammenhang eindeutig ausgeschlossen, ist es grundsätzlich möglich und wird hiermit eindeutig in Betracht gezogen, dass Merkmale unterschiedlicher Ausführungsformen auch in den anderen hierin beschriebenen Ausführungsformen vorhanden sein können. Ebenso wird grundsätzlich erwogen, dass alle Merkmale, die hierin in Zusammenhang mit einem Verfahren beschrieben werden, auch für die hierin beschriebenen Erzeugnisse und Vorrichtungen anwendbar sind, und umgekehrt. Lediglich aus Gründen der knapperen Darstellung werden alle diese erwogenen Kombinationen nicht in allen Fällen explizit aufgeführt. Auch technische Lösungen, die zu den hierin beschriebenen Merkmalen bekanntermaßen gleichwertig sind, sollen grundsätzlich vom Umfang der Erfindung umfasst sein.

Ein erster Aspekt betrifft eine Vorrichtung zur Abgabe einer Flüssigkeit aus einer Glasampulle, aufweisend
- ein Gehäuse,
- eine in dem Gehäuse angeordnete Halterung zur Aufnahme einer Glasampulle,
- einen in dem Gehäuse angeordneten Vorsprung, der zum Aufbrechen einer in der Halterung aufnehmbaren Glasampulle ausgebildet und eingerichtet ist,
- einen Auslass zur Abgabe einer Flüssigkeit aus dem Gehäuse, und
- einen in dem Gehäuse beweglich angeordneten Kolben, wobei der Kolben ausgebildet und eingerichtet ist, eine in der Halterung aufnehmbare Glasampulle durch Druck der Glasampulle gegen den Vorsprung aufzubrechen, und die Flüssigkeit aus der Glasampulle aus dem Auslass des Gehäuses zu pumpen.

Die Vorrichtung weist bevorzugt ein erstes Ende und ein zweites Ende auf, wobei das erste Ende und das zweite Ende bevorzugt gegenüberliegend zueinander angeordnet sind. Das zweite Ende ist bevorzugt eingerichtet, während der Benutzung der Vorrichtung nach unten ausgerichtet zu werden, um die in einer Glasampulle enthaltene Flüssigkeit nach unten abzugeben. "Unten" bezeichnet in diesem Zusammenhang die Richtung der Schwerkraft. "Oben" bezeichnet in entsprechender Weise die entgegengesetzte Richtung zur Schwerkraft. Dementsprechend kann die Vorrichtung bevorzugt eingerichtet sein, während der Benutzung der Vorrichtung mit dem ersten Ende nach oben ausgerichtet zu werden.

Die Vorrichtung weist ein Gehäuse auf, in welchem die einzelnen hierin beschriebenen Elemente der Vorrichtung angeordnet sein können. Das Gehäuse weist einen Innenraum auf, in welchem eine Halterung zur Aufnahme einer Glasampulle angeordnet ist.

Die Vorrichtung ist zur Aufnahme einer Glasampulle eingerichtet. Eine Glasampulle ist ein Behälter aus Glas, welcher eine darin enthaltene Flüssigkeit gasdicht und flüssigkeitsdicht nach außen abgrenzt. Glasampullen haben üblicherweise einen Grundkörper mit im Wesentlichen zylindrischer Form. Die hierin beschriebenen Glasampullen weisen bevorzugt einen Ampullenkopf auf, der durch einen Ampullenhals mit dem übrigen Glaskörper, d. h. dem Grundkörper, der Glasampulle verbunden ist. Der Ampullenhals weist einen niedrigeren Durchmesser als der Grundkörper auf. Der Ampullenhals kann eine Sollbruchstelle aufweisen, wobei der Ampullenkopf durch Abbruch an dieser Sollbruchstelle von der Glasampulle trennbar ist. Die Sollbruchstelle definiert daher eine Öffnung, aus der die Flüssigkeit nach Abbruch des Ampullenkopfs aus der Glasampulle herausfließen kann. Die Sollbruchstelle ist bevorzugt ringförmig. Der Innendurchmesser der Sollbruchstelle kann bevorzugt 4 bis 7 mm betragen, beispielsweise 5 bis 6 mm. Ein Durchmesser der Sollbruchstelle von mehr als 4 mm kann das Herausfließen der in der Glasampulle befindlichen Flüssigkeit erleichtern. Dies ist insbesondere der Fall, wenn die Glasampulle während der Benutzung der hierin beschriebenen Vorrichtung in einem Winkel von 20° bis 50° zur Senkrechten (d. h. in Richtung der Schwerkraft) angeordnet ist. Daher ist in einer Ausführungsform die Vorrichtung eingerichtet, eine Glasampulle in einem Winkel von 20° bis 50° zur Senkrechten aufzubrechen und zu entleeren, wobei der Innendurchmesser der Sollbruchstelle der Glasampulle 4 bis 7 mm, beispielsweise 5 bis 6 mm beträgt.

Die erfindungsgemäßen Vorrichtung weist eine Halterung zur Aufnahme einer solchen Glasampulle auf. Die Halterung kann eine dauerhafte Lagerung einer Glasampulle in der Vorrichtung ermöglichen. Die Halterung kann beispielsweise eingerichtet sein, eine handelsübliche Glasampulle kraftschlüssig oder formschlüssig zu fixieren, sodass die Glasampulle unbeweglich in der Halterung positioniert werden kann. Die Glasampulle kann bevorzugt nach deren Aufnahme in der Halterung nur noch mithilfe des Kolbens innerhalb der Vorrichtung bewegt werden. Hierzu kann die Halterung eine Arretierung zu Fixierung einer Glasampulle aufweisen. Beispielsweise kann die Vorrichtung zum Halten einer Glasampulle mit einer elastischen Nase oder Strebe ausgestattet sein, welche zum Beispiel in Richtung des ersten Endes der Vorrichtung angeordnet sein kann. Die Vorrichtung kann weiterhin einen Kragen aufweisen, der zum Beispiel in Richtung des zweiten Endes der Vorrichtung angeordnet sein kann. Der Kragen kann so dimensioniert sein, dass der Ampullenkopf durch den Kragen geschoben werden kann, wobei der Kragen dem Grundkörper der Ampulle zurückhält. Eine Glasampulle kann beispielsweise zwischen einem solchen Kragen und einer Nase oder Strebe, oder einem vergleichbaren Element zu diesem Zweck, in der Halterung gehalten werden.

Die Vorrichtung weist weiterhin einen Vorsprung auf. Der Vorsprung ist zum Aufbrechen einer in der Halterung aufnehmbaren Glasampulle ausgebildet und eingerichtet. Der Vorsprung kann hierzu derart in dem Gehäuse angeordnet sein, dass er die freie Bewegung einer in der Halterung aufnehmbaren Glasampulle einschränkt. Insbesondere kann der Vorsprung so angeordnet sein, dass der Kopf einer in der Halterung aufnehmbaren Glasampulle mit dem Vorsprung kollidiert, wenn die Glasampulle innerhalb der Vorrichtung in Richtung des zweiten Endes der Vorrichtung bewegt wird. Der Vorsprung kann sich beispielsweise vom Rand des Gehäuses in Richtung der Mitte des Innenraums des Gehäuses erstrecken.

Die Vorrichtung weist weiterhin einen Auslass auf, um Flüssigkeit aus dem Innenraum des Gehäuses nach außen abzugeben. Der Auslass kann eine Öffnung, eine Düse, eine Röhre oder eine Schlauchleitung aufweisen. Der Auslass kann alternativ oder zusätzlich auch einen Adapter zum Anschluss an einen Behälter mit Knochenzementpulver aufweisen.

Die Vorrichtung weist weiterhin einen in dem Gehäuse beweglich angeordneten Kolben auf. Dieser Kolben ist ausgebildet und eingerichtet, eine in der Halterung aufnehmbare Glasampulle durch Druck der Glasampulle gegen den Vorsprung aufzubrechen. Dabei kann der Kolben die Halterung in Richtung des Vorsprung bewegen, um den Kopf der Glasampulle vom Grundkörper der Glasampulle abzubrechen. Weiterhin ist der Kolben ausgebildet und eingerichtet, eine Flüssigkeit aus der wie oben beschrieben aufgebrochenen Glasampulle aus dem Auslass des Gehäuses zu pumpen.

Demnach kann der Kolben zwei Funktionen ausüben, nämlich das Aufbrechen einer in der Vorrichtung aufnehmbaren Glasampulle, und die Abgabe der in einer solchen Glasampulle enthaltenen Flüssigkeit aus der Vorrichtung.

Die Vorrichtung weist bevorzugt weiterhin einen Griff auf, welcher einem Benutzer das manuelle Greifen und Bewegen des Kolbens ermöglicht. Der Griff kann beispielsweise am ersten Ende der Vorrichtung, bevorzugt am gegenüberliegenden Ende zum Auslass der Vorrichtung, angeordnet sein.

In einer bevorzugten Ausführungsform weist die Vorrichtung ein Ventil auf. Das Ventil kann eingerichtet sein, Luft von außen in das Gehäuse einströmen zu lassen. Weiterhin kann das Ventil eingerichtet sein, durch Druck des Kolbens auf die in das Gehäuse eingeströmte Luft eine Flüssigkeit aus einer in der Vorrichtung aufgebrochenen Glasampulle aus dem Gehäuse zu pumpen. Das Ventil kann einen Filter aufweisen, um zu verhindern, dass Staub oder Keime von außen in die Vorrichtung gelangen.

Das Ventil kann bevorzugt ein Einwegeventil sein, d. h. ein Ventil, welches in eine Richtung luftdurchlässig und in eine andere Richtung luftundurchlässig ist. Das Ventil kann beispielsweise ein Kugelventil, Lippenventil oder Plattenventil sein.

Die Vorrichtung kann weiterhin einen Auffangbereich aufweisen. Der Auffangbereich kann in dem Gehäuse angeordnet und zur Aufnahme eines durch Wirkung des Kolbens abgebrochenen Teils einer Glasampulle eingerichtet sein. Der Auffangbereich ist bevorzugt ausreichend groß dimensioniert, sodass der Ampullenkopf einer in der Vorrichtung aufnehmbaren Glasampulle sich um etwa 90° seitlich drehen kann, und die darin enthaltene Flüssigkeit aus dem Ampullenkopf austreten kann, ohne den Ampullenkopf selbst vollständig zu zerbrechen. Beispiele für einen solchen Auffangbereich sind in EP2404864B1 beschrieben. Bevorzugt sind Höhe und Querschnitt des Auffangbereichs mindestens so groß wie die Höhe des Ampullenkopfs bis zur Sollbruchstelle, sodass der Auffangbereich geeignet ist, eine Drehung des abgebrochenen Ampullenkopfs im Auffangbereich zu ermöglichen, wie es in EP2404864B1 ausführlicher beschrieben ist.

In einer Ausführungsform weist die Vorrichtung weiterhin ein Sieb auf, welches angrenzend an den Auffangbereich angeordnet ist. Das Sieb ist bevorzugt eingerichtet, Bruchstücke aus einer in der Vorrichtung aufgebrochenen Glasampulle, beispielsweise einen Ampullenkopf, in der Vorrichtung zurückzuhalten. Hierdurch kann beispielsweise verhindert werden, dass mithilfe der Vorrichtung hergestellter Knochenzement durch Glassplitter verunreinigt wird. Das Sieb kann beispielsweise ein Metalldrahtgeflecht oder ein Netz aus einem Kunststoffgewebe sein. Das Sieb kann auch eine mit zweckmäßig dimensionierten Löchern versehene Scheibe aus Metall oder Kunststoff sein. Weiterhin können offenporige Schaumstoffe wie zum Beispiel Porex verwendet werden. Bevorzugt sollte das Sieb aus einem Material gefertigt sein, welches durch die in einer aufnehmbaren Glasampulle befindliche Flüssigkeit nicht angegriffen wird.

In einer weiteren Ausführungsform weist die Vorrichtung weiterhin einen Sammelbereich auf, um Flüssigkeit aus einer in der Vorrichtung aufgebrochenen Glasampulle aufzunehmen. Der Sammelbereich ist bevorzugt fluidleitend mit der Halterung verbunden, sodass Flüssigkeit aus einer in der Halterung aufnehmbaren Glasampulle in den Sammelbereich fließen kann. Dabei kann die Flüssigkeit bevorzugt aus der Richtung des ersten Endes der Vorrichtung in Richtung des zweiten Endes der Vorrichtung fließen. Der Sammelbereich kann dazu angeordnet und eingerichtet sein, eine Flüssigkeit aus einer in der Vorrichtung aufgebrochenen Glasampulle aufzunehmen und an den Auslass der Vorrichtung zu leiten. Der Sammelbereich kann durch ein wie oben dargestelltes Sieb vom Auffangbereich abgegrenzt sein, wobei das Sieb eine fluidleitende Verbindung zwischen dem Auffangbereich und dem Sammelbereich bilden kann.

In einer Ausführungsform ist die Vorrichtung zum Mischen von Knochenzement ausgebildet und eingerichtet. Bevorzugt weist die Vorrichtung hierzu weiterhin eine Pulverkomponente zur Zubereitung eines Knochenzements auf. Diese Pulverkomponente kann in einem Behälter angeordnet sein, wobei der Behälter über den Auslass mit Flüssigkeit aus einer in der Vorrichtung aufgebrochenen Glasampulle befüllbar sein kann. Der Auslass kann mithilfe eines Adapters mit dem Behälter verbindbar sein, zum Beispiel durch eine Schnapp- oder Schraubverbindung. Zum Mischen der Flüssigkeit und der Pulverkomponente kann weiterhin ein Mischelement, zum Beispiel ein Mischstab, vorgesehen sein, welches die Durchmischung der Flüssigkeit und der Pulverkomponente ermöglicht. Der Mischstab kann einen Handgriff, einen Stab und eine Mischscheibe aufweisen. In einer Ausführungsform ist der Auslass demnach zur Verbindung mit einem Behälter ausgebildet und eingerichtet. Hierbei kann der Behälter bevorzugt zur Aufnahme einer Pulverkomponente eines Knochenzements eingerichtet sein.

In einer Ausführungsform weist die Vorrichtung ein erstes Ende und ein zweites Ende auf, wobei das erste Ende dem zweiten Ende gegenüberliegend angeordnet ist. Bevorzugt ist der Kolben an dem ersten Ende angeordnet. Dies betrifft bevorzugt einen Teil des Kolbens, der einen Griffbereich zum Anfassen und manuellen Bewegen des Kolbens bildet. Dieser Griffbereich wird hierin auch als "Griff' bezeichnet. Bevorzugt ist der Auslass an dem zweiten Ende angeordnet.

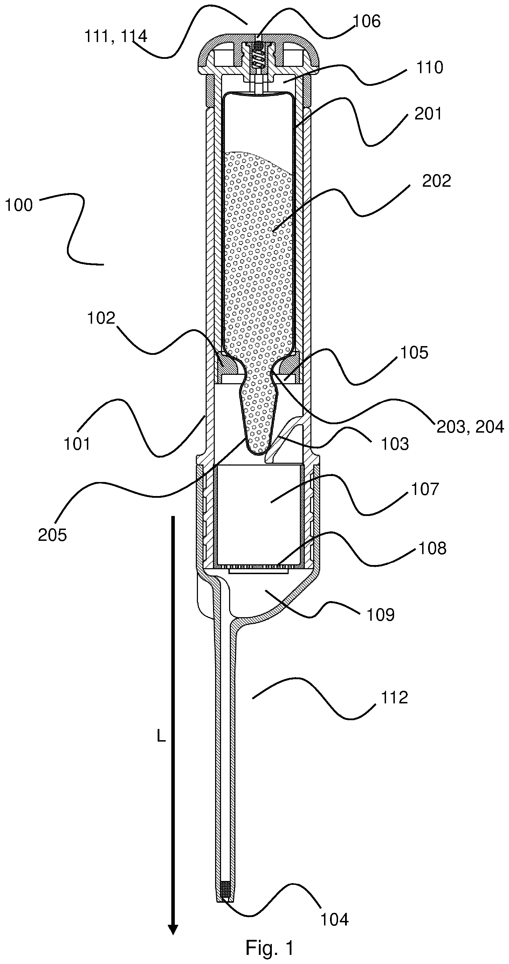

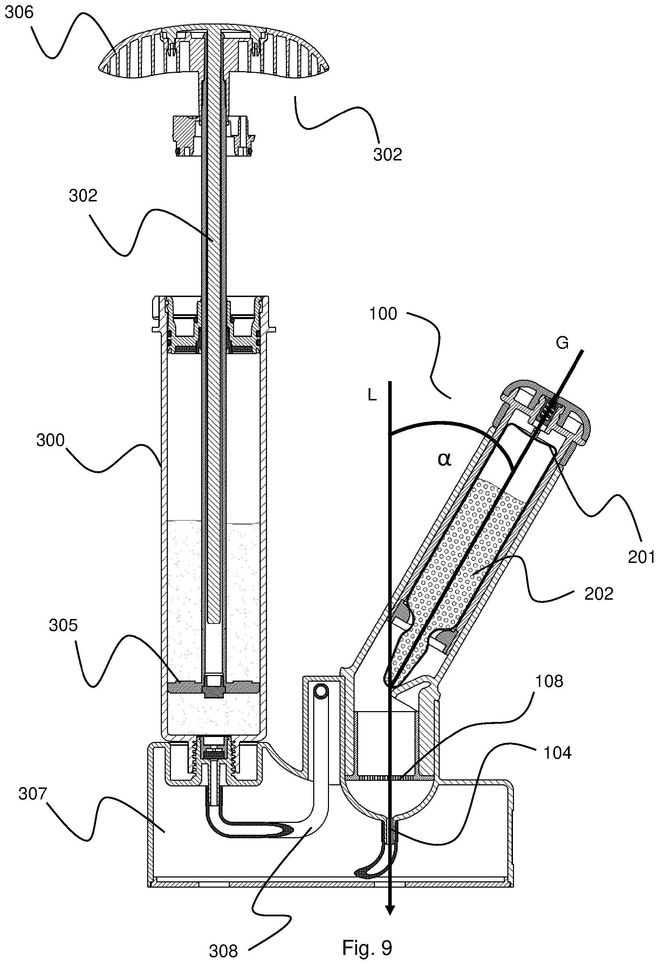

In einer Ausführungsform definiert der Auslass eine Längsachse L der Vorrichtung. Wenn die Vorrichtung, wie beispielsweise in Figur 1 dargestellt, eine im wesentlichen geradlinige Form aufweist, können beispielsweise der Griff und der Auslass in einer Linie liegen, welche die Längsachse L definiert. Wenn die Vorrichtung eine andere Form aufweist, wird die Längsachse L durch die kürzeste Verbindung des Sammelbereichs mit dem Auslasses definiert. Die Vorrichtung ist bevorzugt eingerichtet, eine Flüssigkeit entlang dieser Längsachse L abzugeben. Demnach entspricht die Längsachse L der Richtung, in welcher eine Flüssigkeit im Betrieb der Vorrichtung aus dem Sammelbereich und durch den Auslass fließt. Die Halterung kann bevorzugt in einem Winkel α von 0° bis 50° zur Längsachse L angeordnet sein. Der Winkel α wird von der Längsachse L und einer Geraden G gebildet, welche der Bewegungsrichtung des Kolbens entspricht (siehe hierzu auch beispielhaft Figur 9). Dies ermöglicht es einer Flüssigkeit, aus einer in der Halterung aufgenommenen Glasampulle in den Sammelbereich und durch den Auslass zu fließen, wenn die Vorrichtung mit dem Auslass nach unten (d. h. in Richtung der Schwerkraft) ausgerichtet ist. Der Winkel α kann beispielsweise 0° bis 10°, 0° bis 20°, 0° bis 30°, 10° bis 20°, 15° bis 30°, oder 0° bis 40° betragen.

In einer Ausführungsform ist die Vorrichtung ausgebildet und eingerichtet, mit einem einzigen Hub des Kolbens den gesamten Inhalt einer in der Halterung aufnehmbaren oder aufgenommenen Glasampulle aus dem Auslass des Gehäuses zu pumpen. Dies kann insbesondere dadurch erreicht werden, dass das Hubvolumen der Vorrichtung ausreichend groß im Verhältnis zum Volumen einer abzugebenden Flüssigkeit in der Glasampulle dimensioniert ist, beispielsweise mindestens gleich groß.

In einer Ausführungsform ist eine Glasampulle kraftschlüssig, formschlüssig oder stoffschlüssig in der Halterung befestigt oder fixierbar. Eine Glasampulle kann beispielsweise durch Kleben oder Klemmen in der Vorrichtung fixierbar sein oder befestigt sein. Wie oben dargestellt kann eine Glasampulle beispielsweise im Bereich des Ampullenhalses von einem Kragen gehalten sein, und gleichzeitig am gegenüberliegenden Boden der Ampulle von einer Arretierung, zum Beispiel einer Strebe, einem Vorsprung oder dergleichen, unter Spannung gehalten werden, wie dies beispielhaft in den Figuren veranschaulicht ist.

In einer Ausführungsform ist die die Vorrichtung zum gleichzeitigen Aufbrechen und Abgeben von Flüssigkeit aus zwei Glasampullen ausgebildet und eingerichtet. In einer Ausführungsform ist die Vorrichtung eingerichtet, mithilfe eines einzelnen Kolbens oder mithilfe zweier verbundener Kolben gleichzeitig zwei Glasampullen aufzubrechen und die darin enthaltene Flüssigkeit aus der Vorrichtung abzugeben.

In einer Ausführungsform enthält die Vorrichtung eine Glasampulle. Die Glasampulle kann dabei in der Halterung aufgenommen sein, wie vorangehend erläutert. Die Glasampulle kann eine Flüssigkeit enthalten, beispielsweise eine Monomerlösung zur Herstellung eines Knochenzements, bevorzugt eines PMMA-Knochenzements.

Wenn die Vorrichtung sowohl eine Glasampulle mit einer Flüssigkeit als auch eine zweite Komponente, zum Beispiel eine Flüssigkeit und ein Pulver zur Herstellung von Knochenzement enthält, oder allgemein alle Ausgangsstoffe für den beabsichtigen Zweck der Vorrichtung enthält, kann die Vorrichtung auch als "Full-Prepacked-Mischsystem" bezeichnet werden. Sie ist demnach unmittelbar einsatzbereit zur Herstellung von Knochenzement, ohne weitere Ausgangsstoffe hinzufügen zu müssen. Eine solche Vorrichtung dient bevorzugt zum Lagern der enthaltenen Komponenten sowie zum Mischen und Austragen von Knochenzement, insbesondere Polymethylmethacrylat-Knochenzement. Es sind aber auch andere Anwendungsbereiche im medizinischen oder nichtmedizinischen Gebiet denkbar, in denen eine Flüssigkeit aus einer Glasampulle abgegeben werden soll.

Ein weiterer Aspekt betrifft ein Kit aus einer erfindungsgemäßen Vorrichtung und einer Mischkartusche mit einer Pulverkomponente zur Herstellung von Knochenzement. In einer Ausführungsform weist die Vorrichtung ein Verbindungselement auf, um die Vorrichtung mit der Mischkartusche zu verbinden. Das Verbindungselement kann eine Klemme aufweisen, um das Gehäuse der Vorrichtung mit dem Gehäuse der Mischkartusche zu verbinden. Das Kit kann auch einen Adapter zum Anschluss des Auslass der Vorrichtung an die Mischkartusche aufweisen. Eine Mischkartusche ist ein geschlossener Behälter zur Anmischung von Knochenzement. Die Mischkartusche kann hierzu einen Mischstab enthalten, der eine manuelle oder maschinelle Vermischung der Flüssigkeit und der Pulverkomponente ermöglicht. Der Mischstab kann beispielsweise einen Griff aufweisen, der an der Außenseite des Mischsystems angeordnet sein kann. Der Mischstab kann weiterhin einen Stab umfassen, der den Griff mit einer innerhalb des Mischsystems angeordneten Scheibe verbindet. Die Scheibe kann von einem Benutzer des Mischsystems mithilfe des Griffs innerhalb des Innenraums der hohlzylinderförmigen Kartusche bewegt werden, um hierdurch die Flüssigkeit mit der Pulverkomponente zu mischen. Der Mischstab kann mithilfe einer Verschraubung an der Mischkartusche abgebracht sein.

Ein weiterer Aspekt betrifft ein Verfahren zur Abgabe einer Flüssigkeit aus einer Glasampulle mit Hilfe einer oben beschriebenen Vorrichtung, wobei das Verfahren die folgenden Schritte aufweist,
- Fixieren der Glasampulle in einer Halterung der Vorrichtung;
- Aufbrechen der Glasampulle durch Drücken der Glasampulle gegen einen Vorsprung der Vorrichtung mit Hilfe eines Kolbens der Vorrichtung;
- Abgabe der Flüssigkeit aus der aufgebrochenen Glasampulle durch Pumpen mithilfe des Kolbens durch einen Auslass der Vorrichtung.

Die Flüssigkeit ist bevorzugt eine Monomerflüssigkeit zur Zubereitung eines Knochenzements. Das Verfahren ist bevorzugt ein Verfahren zur Herstellung von Knochenzement. Das Verfahren wird bevorzugt vollständig *in vitro,* d.h. außerhalb des menschlichen Körpers, durchgeführt.

Ein Aspekt der Erfindung betrifft ein Mischsystem, bevorzugt ein Mischsystem für Knochenzement, welches eine hierin beschriebene Vorrichtung enthält. Ein solches Mischsystem kann zwei Komponenten zur Herstellung von Knochenzement enthalten, zum Beispiel eine Monomerflüssigkeit und eine Pulverkomponente zur Herstellung von PMMA-Knochenzement.

Ein beispielhaftes Mischsystem enthält beispielsweise eine hierin beschriebene Vorrichtung, sowie weiterhin eine Mischeinheit, aufweisend eine hohlzylinderförmige Kartusche mit einem Innenraum, wobei in dem Innenraum ein Knochenzementpulver angeordnet ist, und einen Kartuschenkopf, welcher den Innenraum an einem proximalem Kartuschenende fluidleitend verschließt, und ein Leitungsmittel, welches den Innenraum der Mischeinheit fluidleitend mit dem Auslass der Vorrichtung verbindet, wobei sich das Leitungsmittel durch eine Kartuschenkopfdurchführung des Kartuschenkopfs der Mischeinheit erstreckt und das Leitungsmittel und die Kartuschenkopfdurchführung einen Formschluss ausbilden.

Das Mischsystem enthält beispielsweise in einer Ausführungsform
- ein Gehäuse,
- eine in dem Gehäuse angeordnete Halterung zur Aufnahme einer Glasampulle,
- einen in dem Gehäuse angeordneten Vorsprung, der zum Aufbrechen einer in der Halterung aufnehmbaren Glasampulle ausgebildet und eingerichtet ist,
- einen Auslass zur Abgabe einer Flüssigkeit aus dem Gehäuse, und
- einen in dem Gehäuse beweglich angeordneten Kolben, wobei der Kolben ausgebildet und eingerichtet ist, eine in der Halterung aufnehmbare Glasampulle durch Druck der Glasampulle gegen den Vorsprung aufzubrechen, und die Flüssigkeit aus der Glasampulle aus dem Auslass des Gehäuses zu pumpen,
- eine Mischeinheit, aufweisend eine hohlzylinderförmige Kartusche mit einem Innenraum, wobei in dem Innenraum eine Pulverkomponente angeordnet ist,
- einen Kartuschenkopf, welcher den Innenraum an einem proximalem Kartuschenende fluidleitend verschließt,
- und ein Leitungsmittel, welches den Innenraum der Mischeinheit fluidleitend mit dem Auslass der Vorrichtung verbindet, wobei sich das Leitungsmittel durch eine Kartuschenkopfdurchführung des Kartuschenkopfs der Mischeinheit erstreckt und das Leitungsmittel und die Kartuschenkopfdurchführung einen Formschluss ausbilden.

Gemäß dieser Ausführungsform kann mithilfe des Kolbens eine Glasampulle aufgebrochen werden, und die darin enthaltene Flüssigkeit mithilfe eines Leitungsmittels in die Mischeinheit gepumpt werden.

Das Leitungsmittel kann beispielsweise eine Schlauchverbindung sein, welche innerhalb des Mischsystems einzelne Elemente des Mischsystems fluidleitend miteinander verbindet. Das Leitungsmittel kann bevorzugt ein elastisches Polymer aufweisen, oder daraus bestehen. Das elastische Polymer kann beispielsweise ein Silikon, PE, PP, PU, PA oder FEP umfassen.

In einer weiteren Ausführungsform enthält das Mischsystem
- ein Gehäuse,
- eine in dem Gehäuse angeordnete Halterung zur Aufnahme einer Glasampulle,
- einen in dem Gehäuse angeordneten Vorsprung, der zum Aufbrechen einer in der Halterung aufnehmbaren Glasampulle ausgebildet und eingerichtet ist,
- einen Auslass zur Abgabe einer Flüssigkeit aus dem Gehäuse, und
- einen in dem Gehäuse beweglich angeordneten Kolben, wobei der Kolben ausgebildet und eingerichtet ist, eine in der Halterung aufnehmbare Glasampulle durch Druck der Glasampulle gegen den Vorsprung aufzubrechen, und die Flüssigkeit aus der Glasampulle aus dem Auslass des Gehäuses zu pumpen,
- eine Mischeinheit, aufweisend eine hohlzylinderförmige Kartusche mit einem Innenraum, wobei in dem Innenraum eine Pulverkomponente angeordnet ist,
- einen Kartuschenkopf, welcher den Innenraum an einem proximalem Kartuschenende fluidleitend verschließt,
- und einen hohlzylinderförmigen Adapter, der zur reversiblen Verbindung des proximalen Kartuschenendes mit dem Auslass eingerichtet ist.

Gemäß dieser Ausführungsform kann mithilfe des Kolbens eine Glasampulle aufgebrochen werden, und die darin enthaltene Flüssigkeit mithilfe eines reversibel verbindbaren Adapters in die Mischeinheit gepumpt werden.

In einer weiteren Ausführungsform enthält das Mischsystem
- ein Gehäuse,
- eine in dem Gehäuse angeordnete Halterung zur Aufnahme einer Glasampulle,
- einen in dem Gehäuse angeordneten Vorsprung, der zum Aufbrechen einer in der Halterung aufnehmbaren Glasampulle ausgebildet und eingerichtet ist,
- einen Auslass zur Abgabe einer Flüssigkeit aus dem Gehäuse, und
- einen in dem Gehäuse beweglich angeordneten Kolben, wobei der Kolben ausgebildet und eingerichtet ist, eine in der Halterung aufnehmbare Glasampulle durch Druck der Glasampulle gegen den Vorsprung aufzubrechen, und die Flüssigkeit aus der Glasampulle aus dem Auslass des Gehäuses zu pumpen,
- eine Mischeinheit, aufweisend eine hohlzylinderförmige Kartusche mit einem Innenraum, wobei in dem Innenraum eine Pulverkomponente angeordnet ist,
- einen Kartuschenkopf, welcher den Innenraum an einem proximalem Kartuschenende fluidleitend verschließt,
- ein Fußteil, welches als Aufstandsfläche für das Mischsystem eingerichtet ist,
- einen Kartuschenkopf, der mit dem Fußteil verbindbar ist oder verbunden ist,
- eine Einspritzdüse, die im Fußteil angeordnet ist und sich in den Kartuschenkopf erstreckt,
- und ein Leitungsmittel, das mit der Einspritzdüse fluidleitend verbunden ist.

Gemäß dieser Ausführungsform kann mithilfe des Kolbens eine Glasampulle aufgebrochen werden, und die darin enthaltene Flüssigkeit mithilfe eines Leitungsmittels über eine Einspritzdüse in die Mischeinheit gepumpt werden.

Das Mischsystem ist bevorzugt ein "Full-Prepacked-Mischsystem" wie oben beschrieben. Ein solches Mischsystem kann in einer sterilen und hermetisch verschlossenen Verpackung bereitgestellt werden. Auch das Mischsystem selbst und die enthaltenen Knochenzement-Komponenten können dabei steril bereitgestellt werden.

Wie hierin zuvor bereits ausführlicher beschrieben, kann durch die Bewegung des Kolbens eine in der Halterung aufgenommene Glasampulle durch Druck gegen den Vorsprung aufgebrochen werden, um eine darin enthaltene Flüssigkeit, beispielsweise eine Monomerflüssigkeit zur Zubereitung von Knochenzement, aus der Glasampulle freizugeben. Weiterhin kann durch die Bewegung des Kolbens diese Flüssigkeit durch den Auslass und das Leitungsmittel in die Mischeinheit gepumpt werden, und sich dort mit dem dort angeordneten Knochenzementpulver mischen, um aus der Flüssigkeit und der Pulverkomponente einen Knochenzement zu bilden. Hierzu können die Flüssigkeit und die Pulverkomponente weiterhin in der Mischeinheit miteinander vermengt werden. Das Mischsystem kann hierzu einen Mischstab enthalten, der eine manuelle oder maschinelle Vermischung der Flüssigkeit und der Pulverkomponente ermöglicht. Der Mischstab kann beispielsweise einen Griff aufweisen, der an der Außenseite des Mischsystems angeordnet sein kann. Der Mischstab kann weiterhin einen Stab umfassen, der den Griff mit einer innerhalb des Mischsystems angeordneten Scheibe verbindet. Die Scheibe kann von einem Benutzer des Mischsystems mithilfe des Griffs innerhalb des Innenraums der hohlzylinderförmigen Kartusche bewegt werden, um hierdurch die Flüssigkeit mit der Pulverkomponente zu mischen.

Das Mischsystem kann weiterhin ein Sieb aufweisen, um Bruchstücke der aufgebrochenen Ampulle zurückzuhalten, wie zuvor ausführlicher beschrieben.

Das Mischsystem kann weiterhin einen Auffangbereich aufweisen, wie zuvor beschrieben. Der Auffangbereich kann in dem Gehäuse angeordnet und zur Aufnahme eines durch Wirkung des Kolbens abgebrochenen Teils einer Glasampulle eingerichtet sein. Der Auffangbereich ist bevorzugt ausreichend groß dimensioniert, sodass der Ampullenkopf einer in der Vorrichtung aufgebrochenen Glasampulle sich um etwa 90° seitlich drehen kann, und die darin enthaltene Flüssigkeit aus dem Ampullenkopf austreten kann, ohne den Ampullenkopf selbst vollständig zu zerbrechen.

### BEISPIELE

Die Erfindung wird nachfolgend anhand von Beispielen weiter verdeutlicht, die jedoch nicht als einschränkend zu verstehen sind. Dem Fachmann wird ersichtlich sein, dass anstelle der hier beschriebenen Merkmale andere äquivalente Mittel in ähnlicher Weise verwendet werden können.
**Fig. 1** zeigt beispielhaft eine erste Ausführungsform einer erfindungsgemäßen Vorrichtung **100** in einer seitlichen Querschnittsansicht. Die Vorrichtung weist ein erstes Ende **111** auf, welches bestimmungsgemäß während der Benutzung nach oben zeigt, und ein zweites Ende **112**, welches bestimmungsgemäß während der Benutzung nach unten ausgerichtet wird. Die Vorrichtung weist ein Gehäuse **101** mit einem Innenraum **110** auf, indem eine Halterung **102** angeordnet ist. In der Halterung **102** kann eine Glasampulle **201** aufgenommen werden. Die Glasampulle **201** weist einen Ampullenhals **203** mit einer Sollbruchstelle **204** auf, an der der Ampullenkopf **205** abgetrennt werden kann. Die Glasampulle **201** ist mit dem Ampullenkopf in Richtung des zweiten Endes **112** angeordnet und enthält eine Flüssigkeit **202**, zum Beispiel eine Monomerlösung zur Herstellung von Knochenzement. Die Glasampulle **201** ist in einer Halterung **102** gehalten, welche in diesem Beispiel eine kragenförmige Form aufweist. Die Vorrichtung weist am ersten Ende **111** einen Griff **114** auf, der die manuelle Bewegung eines innerhalb des Innenraums **110** angeordneten Kolben **105** ermöglicht. Der Griff **114** ist hierzu operativ mit dem Kolben **105** verbunden. Am ersten Ende **111** weist die Vorrichtung weiterhin ein Ventil **106** auf, welches das Einströmen von Luft in den Innenraum **110** ermöglicht, aber keine Luft aus dem Innenraum **110** nach außen gelangen lässt. Im Innenraum **110** ist weiterhin ein Vorsprung **103** angeordnet, der ein Abbrechen des Ampullenkopf **205** ermöglicht, wenn die Ampulle **201** mithilfe des Kolbens **105** in Richtung des zweiten Endes **112** bewegt wird. Der Vorsprung **103** begrenzt den möglichen Verfahrweg des Kolbens **105** in Richtung des zweiten Endes **112.** Vom Vorsprung **103** in Richtung des zweiten Endes **112** ist weiterhin ein Auffangbereich **107** angeordnet, in dem ein abgebrochener Ampullenkopf **205** aufgenommen werden kann. Der Auffangbereich **107** ist bevorzugt groß genug, um ein seitliches Umkippen des Ampullenkopf **205** zu ermöglichen, sodass die Flüssigkeit **202** aus dem Ampullenkopf **205** auslaufen kann. Der Auffangbereich **107** wird in Richtung des zweiten Endes **112** durch ein Sieb **108** begrenzt, welches Glasbruchstücke einer aufgebrochenen Glasampulle **201** innerhalb der Vorrichtung zurückhalten kann, aber ein Hindurchtreten der Flüssigkeit **202** in einen Sammelbereich **109** erlaubt, der sich angrenzend an das Sieb **108** in Richtung des zweiten Endes **112** befindet. Die Flüssigkeit **202** kann sich im Sammelbereich **109** sammeln, um durch Pumpwirkung des Kolbens **105** aus einem Auslass **104** entlang einer Längsachse der Vorrichtung **L** aus der Vorrichtung abgegeben zu werden.

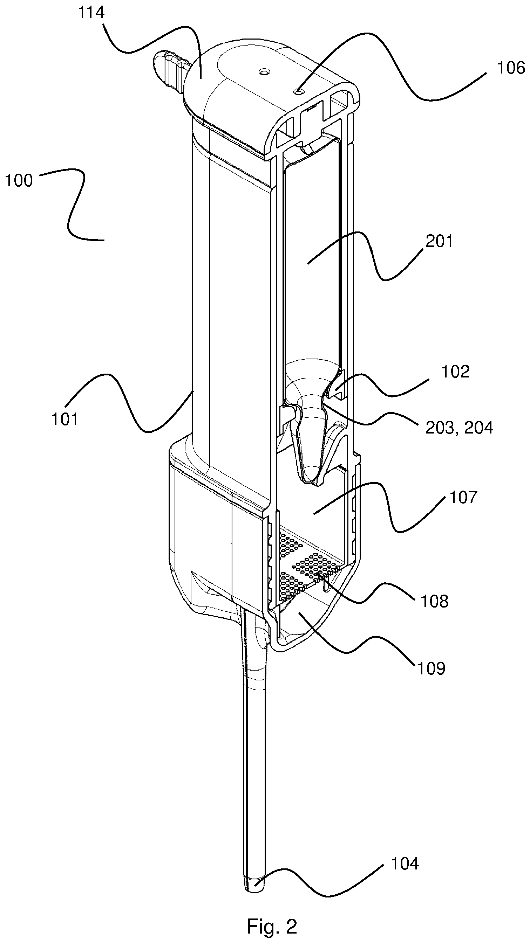

**Fig. 2** zeigt eine Ausführungsform einer erfindungsgemäßen Vorrichtung **100** in einer isometrischen Querschnittansicht. Am Griffbereich **114** sind zwei Ventile **106** angeordnet. Innerhalb des Gehäuses **101** ist eine Glasampulle **201** in einer Halterung **102** angeordnet, wobei der Ampullenhals **203** mit der Sollbruchstelle **204** innerhalb eines Kragens der Halterung **102** gehalten wird. Unterhalb der Glasampulle befindet sich ein Sieb **108**, welches den unmittelbar unterhalb der Ampulle angeordneten Auffangbereich **107** von dem Sammelbereich **109** abtrennt. Der Sammelbereich **109** verjüngt sich zu einem Auslass **104**, welcher hier rohrförmig ausgebildet ist.

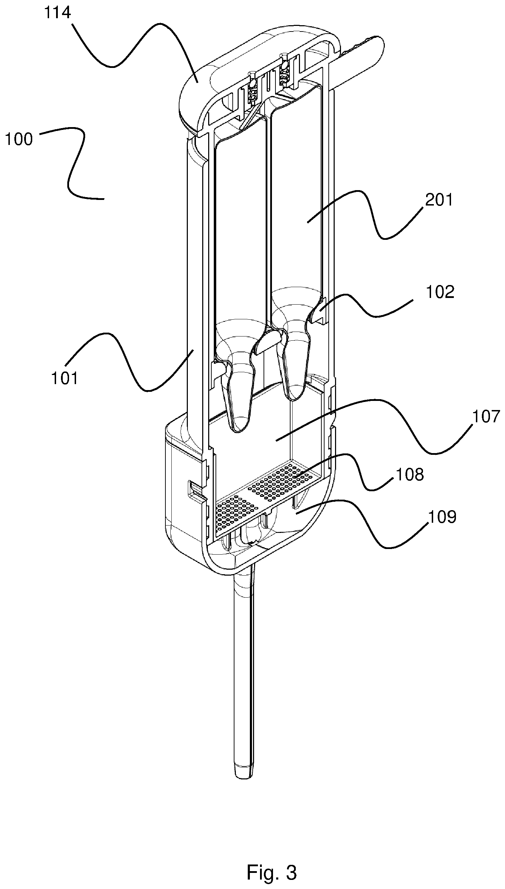

**Fig. 3** zeigt eine weitere Ausführungsform einer erfindungsgemäßen Vorrichtung **100**, welche zum gleichzeitigen Aufbrechen zweier Glasampullen **201** eingerichtet ist. Innerhalb des Gehäuses **101** sind zwei Glasampullen **201** parallel angeordnet, und werden jeweils von einer Halterung **102** gehalten. Unterhalb der Glasampullen **201** befindet sich ein Auffangbereich **107** zur Aufnahme der abgebrochenen Ampullenköpfe der beiden Ampullen **201**.Glasbruchstücke der Ampullen können in einem Sieb **108** unterhalb des Auffangbereich **107** zurückgehalten werden, während die Flüssigkeit aus den beiden Glasampullen durch das Sieb **108** in den Sammelbereich **109** fließen kann.

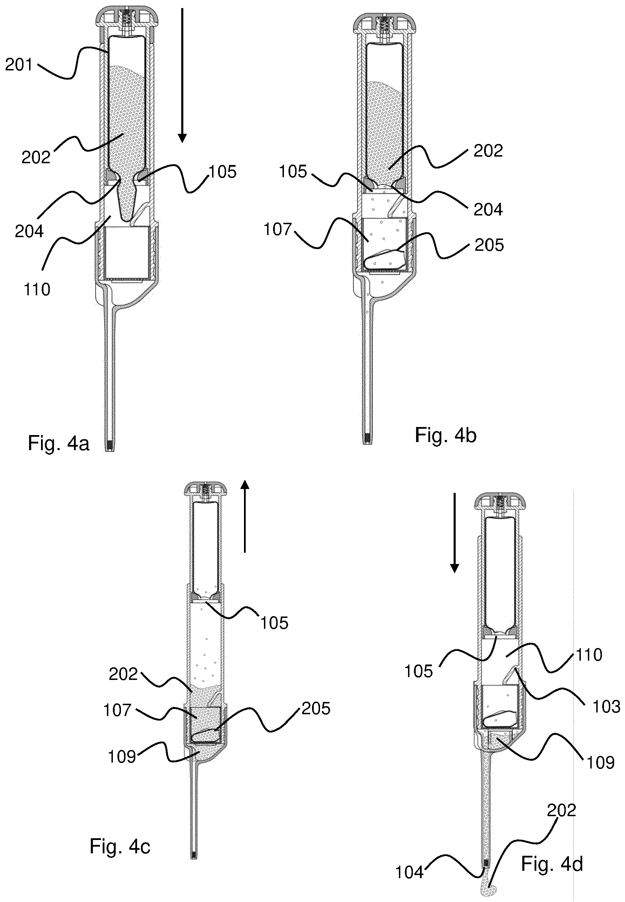

**Figuren 4a bis 4f** zeigen ein Verfahren, bei welchem eine erfindungsgemäße Vorrichtung zur Abgabe einer Flüssigkeit aus einer Glasampulle **201** verwendet wird. Die Bewegungsrichtung des Kolbens ist jeweils durch Pfeile verdeutlicht.
**Fig. 4a** zeigt, wie durch Bewegung des Kolbens **105** eine Glasampulle **201**, die durch eine Halterung **105** innerhalb der Vorrichtung fixiert ist, in Richtung des Vorsprung **103** bewegt wird, um den Ampullenkopf **205** an der Sollbruchstelle **204** abzubrechen.
**Fig. 4b** zeigt den abgebrochenen Ampullenkopf **205**, der in den Auffangbereich **107** gefallen ist, wobei die Öffnung des Ampullenkopfs zur Seite zeigt, um die Flüssigkeit **202** herausfließen zu lassen. Der Kolben **105** ist bis zur Position des Vorsprung **103** in den Innenraum **110** eingeschoben.
**Fig. 4c** zeigt die Vorrichtung in einer Position, in der der Kolben **105** teilweise aus dem Innenraum **110** herausgezogen ist, um den Innenraum **110** mit Luft zu füllen. Die Flüssigkeit **202** der aufgebrochenen Ampulle ist in den Innenraum **110** ausgetreten, insbesondere in den Sammelbereich **109.**
**Fig. 4d** zeigt, wie durch Einschieben des Kolbens **105** in den Innenraum **110** Druck aufgebaut wird, um die Flüssigkeit **202** aus dem Sammelbereich **109** durch den Auslass **104** aus der Vorrichtung auszubringen.

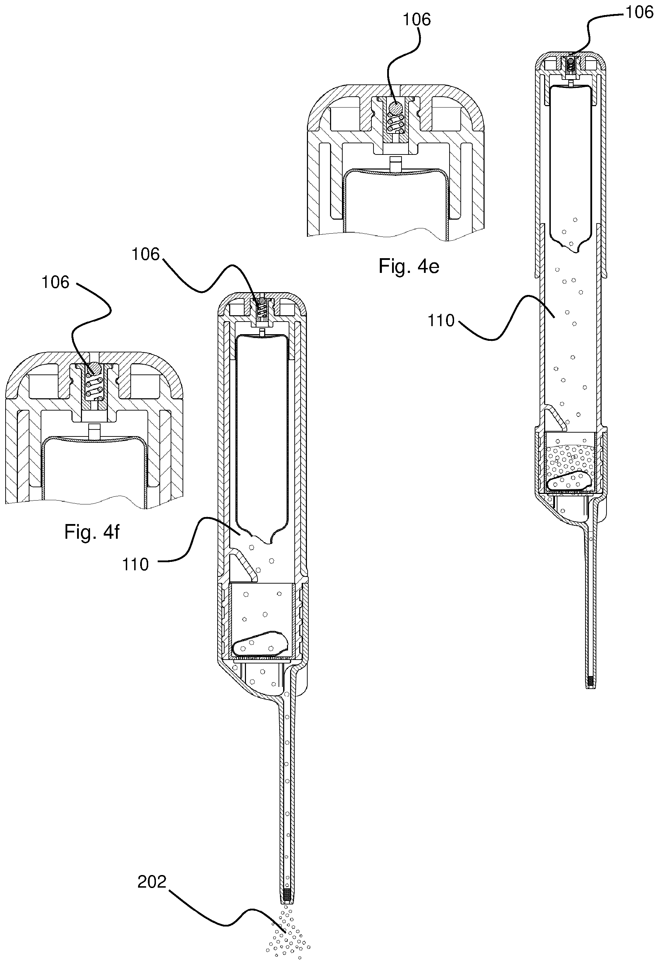

**Fig. 4e** zeigt ein geöffnetes Ventil **106**, während der Kolben **105** aus der Vorrichtung gezogen wird, um Luft in den Innenraum **110** zu pumpen.
**Fig. 4f** zeigt ein geschlossenes Ventil **106**, während der Kolben **105** in die Vorrichtung eingeschoben wird, um Flüssigkeit **202** aus der Vorrichtung herauszupumpen.

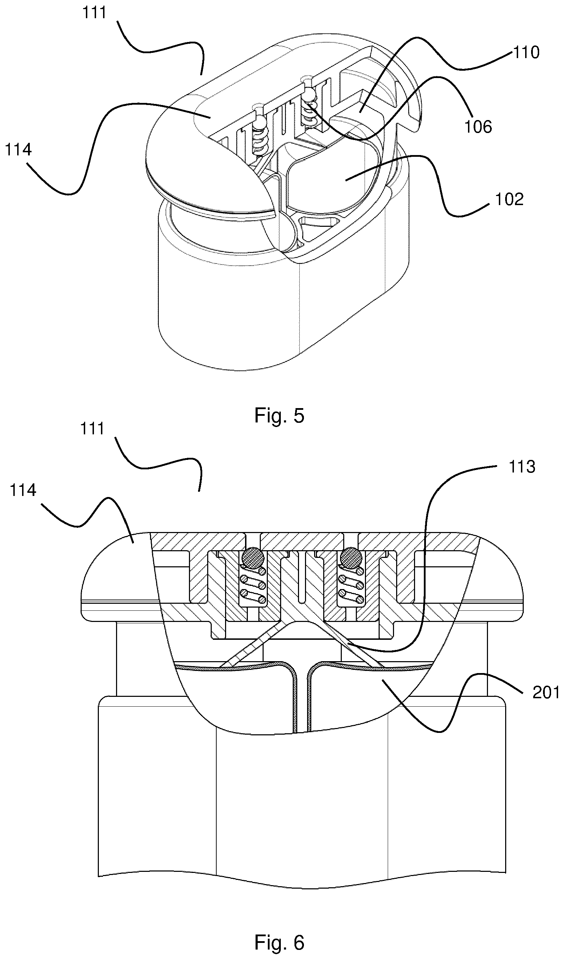

**Fig. 5** zeigt einen Ausschnitt einer erfindungsgemäßen Vorrichtung im Bereich des ersten Endes **111** in einer isometrischen Querschnittansicht. Innerhalb des Griffbereich **114** sind zwei Ventile **106** angeordnet, welche ein Eintreten von Luft in den Innenraum **110** erlauben, in welchem eine Halterung **102** angeordnet ist.
**Fig. 6** zeigt einen Ausschnitt einer erfindungsgemäßen Vorrichtung im Bereich des ersten Endes **111** in einer Querschnittansicht. Angrenzend an den Griffbereich **114** sind Streben **113** als Arretierung für die Glasampullen **201** angeordnet, um diese zu fixieren.

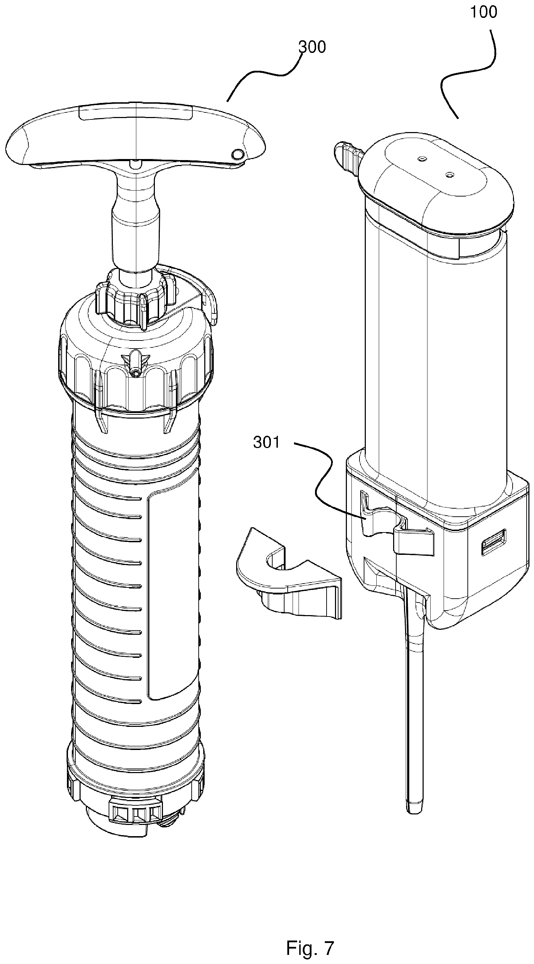

**Fig. 7** zeigt ein Kit aus einer erfindungsgemäßen Vorrichtung **100** und einer Mischkartusche **300** mit einer Pulverkomponente zur Herstellung von Knochenzement. In dieser Ausführungsform weist die Vorrichtung **101** ein Verbindungselement **301** auf, um die Vorrichtung **100** mit der Mischkartusche **300** zu verbinden. Das Verbindungselement ist hier als zweiteilige Klemme ausgebildet, die am Stab des Mischstabs der Mischkartusche angebracht werden kann.

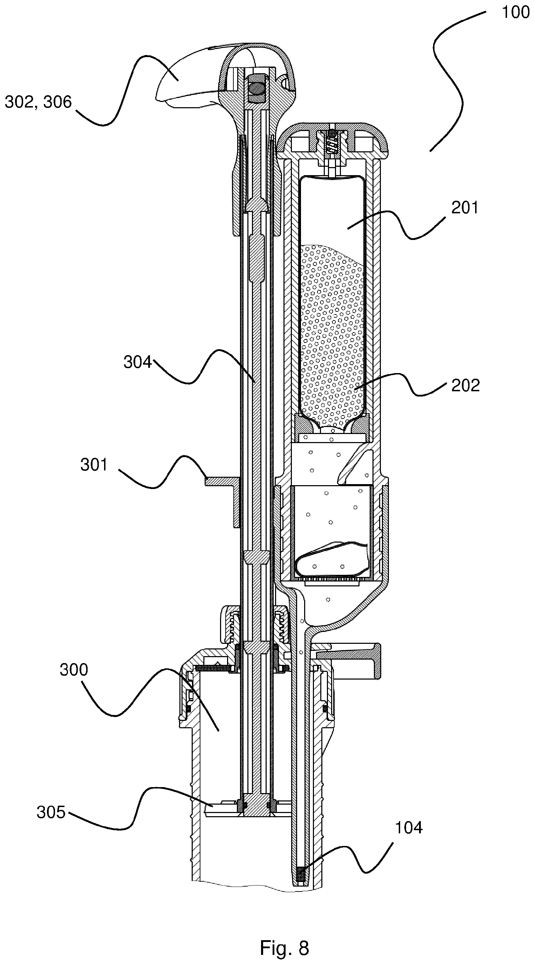

**Fig. 8** **z**eigt eine erfindungsgemäße Vorrichtung **100**, die mithilfe eines Verbindungselements **301** mit einer Kartusche **300** verbunden ist, wobei der Auslass **104** der Vorrichtung **100** in den Innenraum der Kartusche **300** hineinragt, um eine Flüssigkeit **202** aus einer Glasampulle **201** in den Behälter **300** zu pumpen. Die Kartusche **300** weist einen Mischstab **302** mit einem Stab **304**, einem Handgriff **306** und einer Mischscheibe **305** auf. Die Kartusche **300** enthält eine Pulverkomponente zur Herstellung eines PMMA-Knochenzements. Der Mischstab **302** kann von einem Benutzer am Handgriff **306** gehalten und bewegt werden, wobei hierdurch die Mischscheibe **305** innerhalb des Innenraums der Kartusche **300** bewegt werden kann, um die Pulverkomponente mit der Flüssigkeit **201** zu vermischen. Der Griff **306** ist hierfür über einen Stab **304** mit der Mischscheibe **305** verbunden.
**Fig. 9** zeigt eine weitere Ausführungsform eines Mischsystems mit einer erfindungsgemäßen Vorrichtung **100**, welche mit einer Kartusche **300** mit einer Pulverkomponente verbunden ist. Das Mischsystem weist ein Fußteil **307** auf, welches als Aufstandsfläche für das Mischsystem eingerichtet ist, und die Vorrichtung **100** und die Kartusche **300** zueinander in Position hält. Der Auslass **104** der Vorrichtung **100** ist über ein Leitungselement **308** mit dem Innenraum der Kartusche **300** verbunden, um Flüssigkeit **202** aus einer in der Vorrichtung gehaltenen Glasampulle **201** aus der Vorrichtung **100** in den Behälter **300** zu pumpen. Im hier gezeigten Beispiel ist die Ampullenhalterung **102** gewinkelt angeordnet: die Gerade **G**, welche entlang der Mittelachse der Glasampulle verläuft, bildet einen Winkel **α** zur Längsachse **L**, die durch den Auslass **104** und das Sieb **108** definiert wird. Dieser Winkel beträgt etwa 0° bis 50°, sodass sich die Flüssigkeit **202** aus der aufgebrochenen Ampulle **201** durch Wirkung der Schwerkraft in den Sammelbereich **109** bewegen kann. Danach kann die Flüssigkeit **202** durch Bewegung des Kolbens **105** aus der Vorrichtung **100** über den Auslass **104** und das Leitungselement **308** in die Kartusche **300** gepumpt werden. Im hier gezeigten Mischsystem sind die beiden Komponenten für Knochenzement jeweils in getrennten und verschlossenen Räumen eingebracht, nämlich eine Pulverkomponente im Behälter **301** und eine Flüssigkeit **202** in der Vorrichtung **100.** Das Mischsystem ist somit ein Beispiel für ein gebrauchsfertiges "Full-Prepacked-Mischsystem", bei dem der Verwender keine dieser beiden Komponenten zusätzlich hinzufügen muss.

### BEZUGSZEICHENLISTE

- 100: Vorrichtung
- 101: Gehäuse
- 102: Halterung
- 103: Vorsprung
- 104: Auslass
- 105: Kolben
- 106: Ventil
- 107: Auffangbereich (für Glasbruch)
- 108: Sieb
- 109: Sammelbereich (für Flüssigkeit)
- 110: Innenraum des Gehäuses
- 111: erstes Ende
- 112: zweites Ende
- 113: Arretierung (für Glasampulle)
- 114: Griffbereich
- 201: Glasampulle
- 202: Flüssigkeit
- 203: Ampullenhals
- 204: Sollbruchstelle
- 205: Ampullenkopf
- 300: Behälter (für Pulverkomponente)
- 301: Verbindungselement
- 302: Mischstab
- 304: Stab
- 305: Mischscheibe
- 306: Handgriff
- 307: Fußteil
- 308: Leitungselement
- L: Längsachse der Vorrichtung (Abgaberichtung der Flüssigkeit)
- G: Gerade (Mittelachse der Halterung bzw. Ampulle)
- α: Winkel zwischen Längsachse L und Gerade G

## Patentansprüche

1. Vorrichtung (100) zur Abgabe einer Flüssigkeit (202) aus einer Glasampulle (201), aufweisend
- ein Gehäuse (101),
- eine in dem Gehäuse (101) angeordnete Halterung (102) zur Aufnahme einer Glasampulle (201) mit einer Flüssigkeit (202),
- einen in dem Gehäuse (101) angeordneten Vorsprung (103), der zum Aufbrechen einer in der Halterung (102) aufnehmbaren Glasampulle (201) ausgebildet und eingerichtet ist,
- einen Auslass (104) zur Abgabe einer Flüssigkeit (202) aus dem Gehäuse, und
- einen in dem Gehäuse (101) beweglich angeordneten Kolben (105), wobei der Kolben (105) ausgebildet und eingerichtet ist, eine in der Halterung (102) aufnehmbare Glasampulle (201) durch Druck der Glasampulle (201) gegen den Vorsprung (103) aufzubrechen, und die Flüssigkeit (202) aus der Glasampulle (201) aus dem Auslass (104) des Gehäuses (101) zu pumpen.

2. Vorrichtung nach Anspruch 1, weiterhin aufweisend ein Ventil (106), welches eingerichtet ist, Luft von außen in das Gehäuse (101) einströmen zu lassen, um anschließend durch Druck des Kolbens (105) auf die in das Gehäuse eingeströmte Luft eine Flüssigkeit (202) aus einer in der Vorrichtung aufgebrochenen Glasampulle (201) aus dem Gehäuse (101) zu pumpen.

3. Vorrichtung nach Anspruch 2, wobei das Ventil (106) ein Einwegeventil, bevorzugt ein Kugelventil, Lippenventil oder Plattenventil ist.

4. Vorrichtung nach einem der vorangehenden Ansprüche, weiterhin aufweisend einen Auffangbereich (107), der in dem Gehäuse (101) angeordnet und zur Aufnahme eines durch Wirkung des Kolbens (105) abgebrochenen Teils einer Glasampulle (201) eingerichtet ist.

5. Vorrichtung nach Anspruch 4, weiterhin aufweisend ein Sieb (108), welches angrenzend an den Auffangbereich (107) angeordnet ist, und eingerichtet ist, Bruchstücke aus einer in der Vorrichtung aufgebrochenen Glasampulle (201) in der Vorrichtung zurückzuhalten.

6. Vorrichtung nach einem der vorangehenden Ansprüche, wobei die Vorrichtung weiterhin einen Sammelbereich (109) aufweist, um Flüssigkeit (202) aus einer in der Vorrichtung aufgebrochenen Glasampulle (201) aufzunehmen.

7. Vorrichtung nach einem der vorangehenden Ansprüche, wobei die Vorrichtung zum Mischen von Knochenzement ausgebildet und eingerichtet ist, wobei die Vorrichtung bevorzugt weiterhin eine Pulverkomponente zur Zubereitung eines Knochenzements enthält.

8. Vorrichtung nach einem der vorangehenden Ansprüche, wobei die Vorrichtung ein erstes Ende (111) und ein zweites Ende (112) aufweist, wobei das erste Ende (111) dem zweiten Ende (112) gegenüberliegend angeordnet ist, wobei der Kolben (105) an dem ersten Ende (111) angeordnet ist, und der Auslass (104) an dem zweiten Ende (112) angeordnet ist.

9. Vorrichtung nach Anspruch 8, wobei der Auslass (104) eine Längsachse der Vorrichtung (L) definiert, wobei die Vorrichtung eingerichtet ist, eine Flüssigkeit (202) entlang dieser Längsachse abzugeben, wobei die Halterung (102) in einem Winkel α von 0° bis 50° zu dieser Längsachse (L) angeordnet ist.

10. Vorrichtung nach einem der vorangehenden Ansprüche, wobei der Auslass (104) zur Verbindung mit einem Behälter (300) ausgebildet und eingerichtet ist, wobei der Behälter (300) bevorzugt zur Aufnahme einer Pulverkomponente eines Knochenzements eingerichtet ist.

11. Vorrichtung nach einem der vorangehenden Ansprüche, wobei die Vorrichtung ausgebildet und eingerichtet ist, mit einem einzigen Hub des Kolbens (105) den gesamten Inhalt einer in der Halterung (102) aufgenommenen Glasampulle (201) aus dem Auslass (104) des Gehäuses zu pumpen.

12. Vorrichtung nach einem der vorangehenden Ansprüche, wobei eine Glasampulle (201) kraftschlüssig, formschlüssig oder stoffschlüssig in der Halterung (102) befestigt oder fixierbar ist, bevorzugt durch Kleben oder Klemmen befestigt oder fixierbar ist.

13. Vorrichtung nach einem der vorangehenden Ansprüche, wobei die Vorrichtung zum gleichzeitigen Aufbrechen und Abgeben von Flüssigkeit aus zwei Glasampullen ausgebildet und eingerichtet ist.

14. Vorrichtung nach einem der vorangehenden Ansprüche, wobei in der Halterung (102) eine Glasampulle (201) angeordnet ist, die einen Ampullenhals (203) mit einer ringförmigen Sollbruchstelle (204) aufweist, wobei der Innendurchmesser der Sollbruchstelle (204) bevorzugt 4 bis 7 mm beträgt.

15. Verfahren zur Abgabe einer Flüssigkeit (202) aus einer Glasampulle (201) mit Hilfe einer Vorrichtung (100), beispielsweise eine Vorrichtung gemäß einem der Ansprüche 1 bis 14, wobei das Verfahren die folgenden Schritte aufweist,
- Fixieren der Glasampulle (201) in einer Halterung (102) der Vorrichtung;
- Aufbrechen der Glasampulle (201) durch Drücken der Glasampulle (201) gegen einen Vorsprung (103) der Vorrichtung mit Hilfe eines Kolbens (105) der Vorrichtung;
- Abgabe der Flüssigkeit (202) aus der aufgebrochenen Glasampulle durch Pumpen mithilfe des Kolbens (105) durch einen Auslass (104) der Vorrichtung.

## Geänderte Patentansprüche

### Geänderte Patentansprüche gemäss Regel 137(2) EPÜ.

**1.** Vorrichtung (100) zur Abgabe einer Flüssigkeit (202) aus einer Glasampulle (201), aufweisend
- ein Gehäuse (101),
- eine in dem Gehäuse (101) angeordnete Halterung (102) zur Aufnahme einer Glasampulle (201) mit einer Flüssigkeit (202),
- einen in dem Gehäuse (101) angeordneten Vorsprung (103), der zum Aufbrechen einer in der Halterung (102) aufnehmbaren Glasampulle (201) ausgebildet und eingerichtet ist,
- einen Auslass (104) zur Abgabe einer Flüssigkeit (202) aus dem Gehäuse, und
- einen in dem Gehäuse (101) beweglich angeordneten Kolben (105), wobei der Kolben (105) ausgebildet und eingerichtet ist, eine in der Halterung (102) aufnehmbare Glasampulle (201) durch Druck der Glasampulle (201) gegen den Vorsprung (103) aufzubrechen, und die Flüssigkeit (202) aus der Glasampulle (201) aus dem Auslass (104) des Gehäuses (101) zu pumpen,
wobei die Vorrichtung weiterhin ein ein Ventil (106) aufweist, welches eingerichtet ist, Luft von außen in das Gehäuse (101) einströmen zu lassen, um anschließend durch Druck des Kolbens (105) auf die in das Gehäuse eingeströmte Luft eine Flüssigkeit (202) aus einer in der Vorrichtung aufgebrochenen Glasampulle (201) aus dem Gehäuse (101) zu pumpen.

**2.** Vorrichtung nach Anspruch 1, wobei das Ventil (106) ein Einwegeventil, bevorzugt ein Kugelventil, Lippenventil oder Plattenventil ist.

**3.** Vorrichtung nach einem der vorangehenden Ansprüche, weiterhin aufweisend einen Auffangbereich (107), der in dem Gehäuse (101) angeordnet und zur Aufnahme eines durch Wirkung des Kolbens (105) abgebrochenen Teils einer Glasampulle (201) eingerichtet ist.

**4.** Vorrichtung nach Anspruch 3, weiterhin aufweisend ein Sieb (108), welches angrenzend an den Auffangbereich (107) angeordnet ist, und eingerichtet ist, Bruchstücke aus einer in der Vorrichtung aufgebrochenen Glasampulle (201) in der Vorrichtung zurückzuhalten.

**5.** Vorrichtung nach einem der vorangehenden Ansprüche, wobei die Vorrichtung weiterhin einen Sammelbereich (109) aufweist, um Flüssigkeit (202) aus einer in der Vorrichtung aufgebrochenen Glasampulle (201) aufzunehmen.

**6.** Vorrichtung nach einem der vorangehenden Ansprüche, wobei die Vorrichtung zum Mischen von Knochenzement ausgebildet und eingerichtet ist, wobei die Vorrichtung bevorzugt weiterhin eine Pulverkomponente zur Zubereitung eines Knochenzements enthält.

**7.** Vorrichtung nach einem der vorangehenden Ansprüche, wobei die Vorrichtung ein erstes Ende (111) und ein zweites Ende (112) aufweist, wobei das erste Ende (111) dem zweiten Ende (112) gegenüberliegend angeordnet ist, wobei der Kolben (105) an dem ersten Ende (111) angeordnet ist, und der Auslass (104) an dem zweiten Ende (112) angeordnet ist.

**8.** Vorrichtung nach Anspruch 7, wobei der Auslass (104) eine Längsachse der Vorrichtung (L) definiert, wobei die Vorrichtung eingerichtet ist, eine Flüssigkeit (202) entlang dieser Längsachse abzugeben, wobei die Halterung (102) in einem Winkel α von 0° bis 50° zu dieser Längsachse (L) angeordnet ist.

**9.** Vorrichtung nach einem der vorangehenden Ansprüche, wobei der Auslass (104) zur Verbindung mit einem Behälter (300) ausgebildet und eingerichtet ist, wobei der Behälter (300) bevorzugt zur Aufnahme einer Pulverkomponente eines Knochenzements eingerichtet ist.

**10.** Vorrichtung nach einem der vorangehenden Ansprüche, wobei die Vorrichtung ausgebildet und eingerichtet ist, mit einem einzigen Hub des Kolbens (105) den gesamten Inhalt einer in der Halterung (102) aufgenommenen Glasampulle (201) aus dem Auslass (104) des Gehäuses zu pumpen.

**11.** Vorrichtung nach einem der vorangehenden Ansprüche, wobei eine Glasampulle (201) kraftschlüssig, formschlüssig oder stoffschlüssig in der Halterung (102) befestigt oder fixierbar ist, bevorzugt durch Kleben oder Klemmen befestigt oder fixierbar ist.

**12.** Vorrichtung nach einem der vorangehenden Ansprüche, wobei die Vorrichtung zum gleichzeitigen Aufbrechen und Abgeben von Flüssigkeit aus zwei Glasampullen ausgebildet und eingerichtet ist.

**13.** Vorrichtung nach einem der vorangehenden Ansprüche, wobei in der Halterung (102) eine Glasampulle (201) angeordnet ist, die einen Ampullenhals (203) mit einer ringförmigen Sollbruchstelle (204) aufweist, wobei der Innendurchmesser der Sollbruchstelle (204) bevorzugt 4 bis 7 mm beträgt.

**15.** Verfahren zur Abgabe einer Flüssigkeit (202) aus einer Glasampulle (201) mit Hilfe einer Vorrichtung (100)gemäß einem der Ansprüche 1 bis 14, wobei das Verfahren die folgenden Schritte aufweist,
- Fixieren der Glasampulle (201) in einer Halterung (102) der Vorrichtung;
- Aufbrechen der Glasampulle (201) durch Drücken der Glasampulle (201) gegen einen Vorsprung (103) der Vorrichtung mit Hilfe eines Kolbens (105) der Vorrichtung;
- Abgabe der Flüssigkeit (202) aus der aufgebrochenen Glasampulle durch Pumpen mithilfe des Kolbens (105) durch einen Auslass (104) der Vorrichtung.
